(19) Europäisches Patentamt
European Patent Office
Office européen des brevets

(11) **EP 4 129 078 A1**

(12) **EUROPEAN PATENT APPLICATION**
published in accordance with Art. 153(4) EPC

(43) Date of publication:
**08.02.2023 Bulletin 2023/06**

(21) Application number: **21775409.2**

(22) Date of filing: **26.03.2021**

(51) International Patent Classification (IPC):
**A23K 10/16** (2016.01)   **A23K 10/30** (2016.01)
**A23K 20/153** (2016.01)

(52) Cooperative Patent Classification (CPC):
**A23K 10/16; A23K 10/30; A23K 20/153**

(86) International application number:
**PCT/JP2021/012817**

(87) International publication number:
**WO 2021/193907 (30.09.2021 Gazette 2021/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **26.03.2020 JP 2020055577**

(71) Applicant: **Nippon Paper Industries Co., Ltd.
Tokyo 114-0002 (JP)**

(72) Inventors:
• **HASHIMOTO, Tadafumi**
  **Tokyo 114-0002 (JP)**
• **SUGIYAMA, Shinji**
  **Tokyo 114-0002 (JP)**
• **FUJIWARA, Kazuhiro**
  **Tokyo 114-0002 (JP)**

(74) Representative: **Hoffmann Eitle
Patent- und Rechtsanwälte PartmbB
Arabellastraße 30
81925 München (DE)**

(54) **FEED COMPOSITION AND PRODUCTION METHOD THEREFOR**

(57)     One of problems is to provide a feed composition having both immunostimulatory action and mold toxin adsorbing action. Another one of the problems is to provide a technique for minimizing decomposition of a feed composition made using yeast. Yet another one of the problems is to provide a method of inexpensively manufacturing the above-mentioned feed composition.

The feed composition is obtained by inclusion of nucleic acid having molecular weights ranging from 5,000 to 100,000, a yeast cell wall component/yeast cell wall components, and ligno sulfonic acid. A feed composition may be manufactured by supplying nucleic acid and a yeast cell wall component that are both derived from a raw material, yeast. To supply the nucleic acid and a yeast cell wall component/yeast cell wall components from the yeast, the yeast may be subjected to alkaline treatment to prepare the nucleic acid and enucleated yeast.

[FIG. 1]

EP 4 129 078 A1

## Description

**Technical Field**

The present invention relates to feed compositions and manufacturing methods therefor, and particularly relates to feed compositions made using yeast and manufacturing methods therefor.

**Background Art**

Components included in yeast are useful as feed and are used as feed or feed additives. For example, use of yeast as a raw material and use of yeast extract compositions as feed additives have been disclosed, the yeast extract compositions having feeding stimulating action and immunity enhancing action (for example, Patent Literature 1). In addition, feed added with an immunostimulator has been disclosed, the immunostimulator including given amounts of glucans and lipids that are both derived from brewery yeast (for example, Patent Literature 2). Furthermore, feed contaminated with mycotoxins, which are metabolites of molds, may cause problems in growth of animals and cause toxicity problems for animals, and methods of adsorbing and inactivating mycotoxins by use of yeast cell wall extracts have thus been disclosed (for example, Patent Literature 3).

**Citation List**

**Patent Literature**

Patent Literature 1: Japanese Patent Application Laid-open No. H07-184595
Patent Literature 2: Japanese Patent No. 6530846
Patent Literature 3: Japanese National Publication of International Patent Application No. 2002-512011

**Summary of Invention**

Technical Problem

However, feed simultaneously satisfying both an immunizing effect and a mold toxin adsorbing effect has not been found thus far. Furthermore, prior to reduction of mold toxicity by adsorption of mold toxins, enabling minimization of decomposition of feed made using yeast is even more preferable.
Feed is generally desired to be inexpensive and there is a demand for low-cost manufacture of feed having plural kinds of efficacy.
In view of the above circumstances, one of problems to be solved by the present invention is to provide a feed composition having both immunostimulatory action and mold toxin adsorbing action.
Another one of the problems to be solved by the present invention is to provide a technique for conveniently minimizing decomposition of a feed composition made using yeast.
Yet another one of the problems to be solved by the present invention is to provide a method of inexpensively manufacturing the above-mentioned feed composition.

**Solution to Problem**

Inventors of the present invention conducted diligent research; and as a result, have found that inclusion of ligno sulfonic acid, which may be used as a component in a culture medium for culture of yeast, achieves provision of a feed composition that does not decompose easily, in addition to merits of adding components derived from yeast, and have completed the invention. The following compositions for feed and methods of manufacturing the compositions are provided as some of aspects of the present invention.

[1] A feed composition, comprising:

nucleic acid having molecular weights ranging from 5,000 to 100,000;
a yeast cell wall component/yeast cell wall components; and
ligno sulfonic acid.

[2] The feed composition according to the above item [1], wherein the content of ligno sulfonic acid ranges from 1 to 30 wt%.

[3] The feed composition according to the above item [1} or [2], wherein the nucleic acid is ribonucleic acid derived from yeast.

[4] The feed composition according to any one of the above items [1] to [3], further comprising sulfite.

[5] A method of manufacturing a feed composition, the method comprising:

mixing nucleic acid having molecular weights ranging from 5,000 to 100,000, a yeast cell wall component/yeast cell wall components, and ligno sulfonic acid.

[6] The method of manufacturing a feed composition, according to the above item [5], comprising:

collecting nucleic acid and enucleated yeast by separating yeast into the nucleic acid and the enucleated yeast through enucleation of the yeast, thus respectively preparing the nucleic acid having the molecular weights ranging from 5,000 to 100,000 and the yeast cell wall component/yeast cell wall components.

[7] A method of manufacturing a feed composition, the composition comprising nucleic acid having molecular weights ranging from 5,000 to 100,000, a yeast cell wall component/yeast cell wall components, and ligno sulfonic acid, the method comprising:

culturing yeast in a culture medium including ligno sulfonic acid;
subjecting the yeast to alkaline treatment; and
collecting a mixture including: the culture medium including ligno sulfonic acid; nucleic acid derived from the yeast; and a yeast cell wall component/yeast cell wall components derived from the yeast.

**Advantageous Effects of Invention**

According to an embodiment of the present invention, a feed composition made using yeast as a raw material is able to be provided, the composition having immunostimulatory action and mold toxin adsorbing action and not decomposing easily.

Furthermore, according to an embodiment of the present invention, a feed composition is able to be manufactured inexpensively, the feed composition having immunostimulatory action and mold toxin adsorbing action and not decomposing easily.

**Brief Description of Drawings**

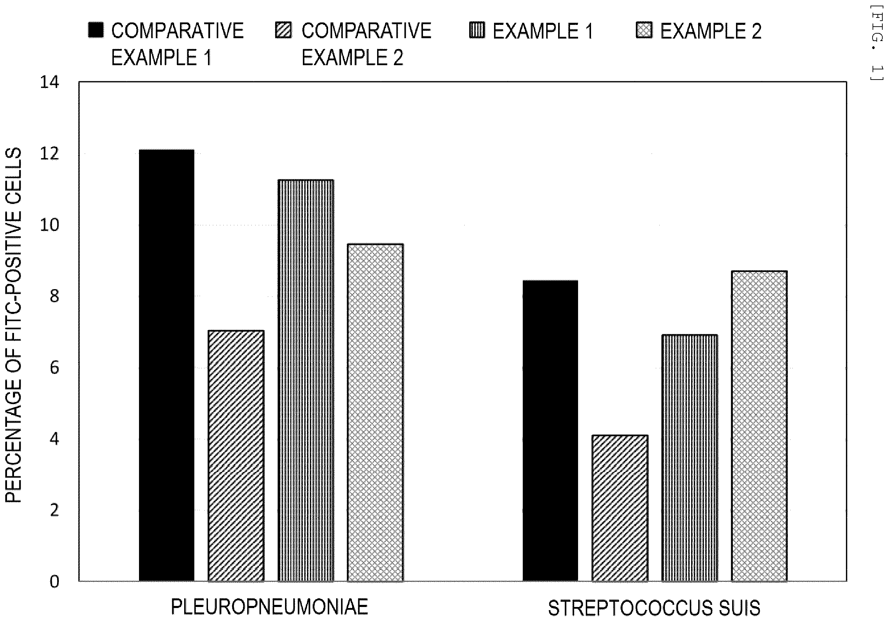

FIG. 1 is a diagram of a graph illustrating results of measurement of phagocytotic activity of macrophages for pathogens. The graph illustrates results of Comparative Examples 1 and 2 and Examples 1 and 2 for each of a section where pleuropneumoniae were used as an antigen and a section where Streptococcus suis was used as an antigen. Bars in the graph represent, from the left, Comparative Example 1 (in black), Comparative Example 2 (with positively sloped lines), Example 1 (with vertical lines), and Example 2 (with grid lines), for each of these sections.

**Description of Embodiments**

Embodiments of the present invention will be described hereinafter. The phrase, "ranging from AA to BB," related a numerical range means "being in the range of AA or more and BB or less" (where "AA" and "BB" represent any numerical values), unless otherwise specified. Furthermore, the units of the lower limit and the upper limit are the same as the unit written immediately after the upper limit (that is, "BB" herein), unless otherwise specified.

1. Feed Compositions

An embodiment of the present invention may be a feed composition. In an embodiment of the present invention, the feed composition contains nucleic acid having molecular weights ranging from 5,000 to 100,000, a yeast cell wall component/yeast cell wall components, and ligno sulfonic acid.

Uses of the feed composition include various aspects used in relation to feed, and for example, may be feed itself to be directly fed to targeted animals, such as livestock, poultry, and fish, or may be a feed additive to be added to other main nutritional components used as feed, such as grain.

In an embodiment of the present invention, a feed composition contains nucleic acid. Including the nucleic acid in the feed composition enables activation of immunity of animals that have ingested the feed.

The nucleic acid may include, not only nucleic acid as being polymers, but also nucleotides that are constituent units of the nucleic acid, and the nucleic acid preferably have molecules that are large to some extent. In a preferred embodiment, for example, a feed composition includes nucleic acid molecules having molecular weights ranging from 5,0000 to 100,000.

The lower limit of the molecular weights of the nucleic acid may be preferably 5,000 or higher, more preferably 6,000, 7,000, 8,000, or 9,000 or higher, and even more preferably 10,000, 15,000, or 20,000 or higher.

The upper limit of the molecular weights of the nucleic acid may be preferably 100,000 or lower, more preferably 80,000 or lower, and even more preferably 70,000, 60,0000, or 50,000 or lower.

A molecular weight distribution of the nucleic acid herein can be found by, for example, gel permeation chromatography (GPC).

In another embodiment of the present invention, a weight-average molecular weight (Mw) may serve as an index for preferred nucleic acid.

The lower limit of the weight-average molecular weight of the nucleic acid may be preferably 5,000 or higher, more preferably 10,000 or higher, and even more preferably 20,000 or higher.

The upper limit of the weight-average molecular weight of the nucleic acid may be preferably 100,000 or lower, more preferably 70,000 or lower, and even more preferably 50,000 or lower.

Types of sugar composing the nucleic acids may be any of deoxyribose and ribose. That is, the nucleic acids may be any of deoxyribonucleic acids (DNAs) and ribonucleic acids (RNAs). Examples of type of bases composing the nucleic acids may mainly include adenine, guanine, thymine, cytosine, and uracil, that is, examples of types of nucleosides composing the nucleic acids may include adenosine, guanosine, cytidine, uridine, and thymidine. Phosphoric acids composing the nucleosides may include mono-phosphoric acid or two or more of phosphoric acids. Commercially available nucleic acids may also be used. One type of nucleic acid may be included alone or a mixture of plural types of nucleic acids may be included. Ribonucleic acids and nucleotides are preferably used as the nucleic acids.

Derivation of the nucleic acid is not particularly limited, and the nucleic acid thus may be artificially synthesized or derived from natural products. For example, nucleic acid extracted or refined from microorganisms, such as yeast, may be used. Such nucleic acid that has been synthesized, extracted, or refined can be made into forms that are easily absorbed when ingested by targeted organisms, such as animals fed with the feed composition. Microorganisms, such as yeast, may be grown using wood sugar to obtain nucleic acid, the wood sugar being included in biological resources that have become waste materials, for example, in lumber that has become scrap lumber; the nucleic acid obtained may be used by being included in a feed composition; and as a result, what have been regarded as waste materials are able to be converted into useful materials and this conversion can contribute to formation of a sustainable recycling-oriented society.

The content of the nucleic acid in the feed composition may be adjusted as appropriate according to the use of the feed composition. For example, the content for feed and the content for an additive for feed would generally be different from each other. The following is examples of the content of the nucleic acid in a case where the feed composition is used as feed.

The lower limit of the content of the nucleic acid in the feed composition may be preferably 3 wt% or higher, more preferably 5, 6, or 7 wt% or higher, and even more preferably 8, 9, or 10 wt% or higher.

The upper limit of the content of the nucleic acid in the feed composition may be preferably 50 wt% or lower, more preferably 30 wt% or lower, and even more preferably 20 wt% or lower.

A feed composition including nucleic acid having the above-mentioned preferred molecular weights (for example, ranging from 5,000 to 100,000) at the preferred content mentioned herein may be an example of a preferred embodiment. In this case, complete exclusion of nucleic acid molecules having molecular weights other than the above-mentioned preferred molecular weights is not required; but the content of these nucleic acid molecules is preferably low and specifically, may be preferably less than the content of nucleic acid molecules having the above-mentioned preferred molecular weights, and may be more preferably 1 wt% or lower.

In an embodiment of the present invention, the feed composition contains a yeast cell wall component/yeast cell wall components. These yeast cell wall components are capable of adsorbing mold toxins. Even if an animal ingests the feed composition that happens to have unwanted bacteria that have grown slightly proliferously, the feed composition may contribute to reduction of absorption, by the animal, of toxins generated from the bacteria and to excretion of the toxins from the animal, due to the mold toxin adsorbing action of the yeast cell wall components.

The term, "a yeast cell wall component/yeast cell wall components," refer to part of or the whole of cell walls derived from yeast, or fiber components derived from yeast. The yeast cell wall components may be enucleated yeast obtained by enucleation for removal of nucleic acid from yeast cells, may be cell walls maintaining the shapes of coats of yeast, or may be cell walls that have been crushed to an extent where the shapes of the coats are not maintained. The cell wall components used in the present invention may be part of yeast cell walls, but desirably include at least fiber remaining in the cell wall components. Inclusion of at least fiber remaining therein enables the cell wall components to have excellent mold toxin adsorption performance. In contrast, if decomposition progresses to an extent where no fiber remains, the mold toxin adsorption performance tends to be reduced.

The content of the yeast cell wall components in the feed composition may be adjusted as appropriate according to the use of the feed composition. For example, the content of the yeast cell wall components for feed and the content of the yeast cell wall components for an additive for feed would generally be different from each other. The following is examples of the content of the yeast cell wall components in a case where the feed composition is used as feed.

The lower limit of the content of the yeast cell wall components in the feed composition may be preferably 3 wt% or higher, more preferably 5 wt% or higher, and even more preferably 10 wt% or higher.

The upper limit of the content of the yeast cell wall components in the feed composition may be preferably 30 wt% or lower, more preferably 25 wt% or lower, and even more preferably 20 wt% or lower.

Yeast may be used as a raw material for the nucleic acid and the yeast cell wall components in the feed composition. Types of yeast that may be used may be sporogenous yeasts or asporogenous yeasts. Specifically, examples of yeast may include the following types.

Examples of the sporogenous yeasts may include yeast of Shizosaccharomyces genus, Saccharomyces genus, Kluyveromyces genus, Hansenula genus, Pichia genus, Debaryomyces genus, and Lipomyces genus, and more specifically, may include: Shizosaccharomyces pombe, Shizosaccharomyces octosporus; Saccharomyces cerevisiae, Saccharomyces uvarum, Saccharomyces rouxii; Kluyveromyces fragilis, Kluyveromyces lactis; Hansenula anomala; Pichia membranaefaciens; Debaryomyces hansenii; and Lipomyces starkeyi.

Examples of the asporogenous yeasts may include yeast of Torulopsis genus, Candida genus, and Rhodotorula genus, and more specifically, may include: Torulopsis versatilis; Candida tropicalis, Candida lipolytica, Candida utilis; and Rhodotorula glutinis.

Yeast of Candida genus is also called torula yeast taxonomically and may be classified as yeast of Cyberlindnera genus.

Examples of the yeast that may be used may preferably include brewery yeast, wine yeast, bakery yeast, and torula yeast, and more specifically, may include:

Saccharomyces cerevisiae, Saccharomyces uvarum,
Saccharomyces rouxii; Kluyveromyces fragilis, Torulopsis versatilis, Candida tropicalis, Candida lipolytica, Candida utilis, and Rhodotorula glutinis.

Candida utilis may be taxonomically classified as a type of torula yeast, Cyberlindnera jadinii.

In an embodiment of the present invention, the feed composition contains ligno sulfonic acid. Including ligno sulfonic acid enables minimization of decomposition of the feed composition made using the raw material, yeast.

Ligno sulfonic acid is a compound having a skeleton, in which carbon at an $\alpha$-position of a side chain in a hydroxyphenyl propane structure of lignin has been cleaved and a sulfo group has been introduced. Ligno sulfonic acid may be in the form of a salt. A lignin sulfonate may be added as ligno sulfonic acid to be added to the feed composition. Examples of the lignin sulfonate may include a calcium salt, a magnesium salt, a sodium salt, a mixture of calcium and sodium salts, an ammonium salt, and an organic ammonium salt. Ligno sulfonic acid may be obtained from, for example, sulfite pulp waste liquor produced in the paper manufacturing industry. Furthermore, ligno sulfonic acid used in the present invention may be ligno sulfonic acid that has been denatured by a polyelectrolyte having a functional group, such as a sulfonic group, a carboxyl group, or a phenolic hydroxyl group.

The content of ligno sulfonic acid in the feed composition may be adjusted as appropriate according to the use of the feed composition. For example, the content of ligno sulfonic acid for feed and the content of ligno sulfonic acid for an additive for feed would generally be different from each other. The following is examples of the content of ligno sulfonic acid in a case where the feed composition is used as feed, although defining the content in a single uniform manner is difficult.

The lower limit of the content of ligno sulfonic acid in the feed composition may be preferably 1 wt% or higher, more preferably 3 wt% or higher, and even more preferably 5 wt% or higher.

The upper limit of the content of ligno sulfonic acid in the feed composition may be preferably 50 wt% or lower, more preferably 40 wt% or lower, and even more preferably 30 wt% or lower.

Another preferred embodiment of the feed composition may include a sulfite. Including a sulfite may contribute to minimization of oxidation or minimization of growth of unwanted bacteria.

In addition, the feed composition may include any optional component/components, as necessary, such as water, oil, a pH adjuster, an antioxidant, a preservative, a coloring material, a fragrance, a diluent, vitamins, hormones, amino acids, an antibiotic, and/or an antimicrobial agent.

The feed composition may have a form generally adopted as feed or an additive for feed. Examples of the form of the feed composition may include a powdery form, a granular form, a mashed form, a pelletized form, a crumbled form, and a flakey form. The feed composition may have a single form or a mixed form of two or more of the forms mentioned above, for example, a mixture of pellets and flakes or a mixture of mash and pellets.

The feed composition may be used, for example, as feed or a feed additive.

In a case of using as feed, a mixture including the above-mentioned nucleic acid, yeast cell wall components, and ligno sulfonic acid may be used as is.

Furthermore, in a case where the feed composition is used as feed, main nutritional components (hereinafter, also referred to as main feed components) may be included in the feed composition. The main feed components may be plant-based feed and/or animal-based feed. The plant-based feed is feed derived from plants, and examples of the plant-based feed may include corn, milo, barley, wheat, cassava, rice bran, bran, soybean cake, rapeseed residue, rice, rice bran, and beet, as well as a processed product of any of these types of plant-based feed. In addition, the animal-based feed is feed derived from animals, and examples of the animal-based feed may include fish meal, pork meal, chicken meal, powdered skim milk, and condensed whey. One of these types of feed may be used alone or any of these types of feed may be used in combination.

Furthermore, in a case where the feed composition is used as an additive for feed, the feed composition may be added as an additive to the above-mentioned main feed components.

2. Methods of Manufacturing Feed Composition

Some of embodiments of the present invention include methods of manufacturing the above-described compositions for feed.

First Embodiment Related to Manufacturing Method

A first embodiment of a manufacturing method according to the present invention includes mixing nucleic acid having molecular weights ranging from 5,000 to 100,000, a yeast cell wall component/yeast cell wall components, and ligno sulfonic acid.

As long as these three kinds of components, the nucleic acid, the yeast cell wall component/yeast cell wall components, and ligno sulfonic acid, are ultimately mixed together, these kinds of components may be mixed one by one or may be added and mixed together at once. The nucleic acid, the yeast cell wall components, and ligno sulfonic acid correspond to those described above. Each of these kinds of components may be individually prepared, or yeast may be cultured and the nucleic acid and yeast cell walls may be prepared from the cultured yeast serving as a raw material.

For example, in a modified example of the first embodiment, nucleic acid and enucleated yeast may be collected by separating yeast into the nucleic acid and the enucleated yeast through enucleation of the yeast, thus respectively preparing nucleic acid having molecular weights ranging from 5,000 to 100,000 and yeast cell wall components. A feed composition is able to be obtained by adding ligno sulfonic acid, in addition to the nucleic acid and yeast cell wall components that have both been prepared as described above.

The enucleation may be conducted by, for example, a method of causing an alkaline agent, saline solution, or cell wall lytic enzyme to be in contact with the yeast to lyse cell walls of the yeast, to cause the contents inside the yeast to elute into a culture medium and separate the yeast into cell wall components and other components. These separated components may be refined and/or pulverized, as necessary. In an embodiment of the present invention, in a case where nucleic acid and yeast cell wall components are to be included, cells of yeast may be added as is, but the nucleic acid and yeast cell wall components are preferably combined back together after being separated each from yeast first for enhancement of the immunostimulatory effect.

An ordinary method used for obtainment of a yeast extract may be used as a method of alkaline treatment. That is, part or all of cell walls of yeast may be lysed or broken by use of an alkaline agent to cause components in the yeast cells to elute. Examples of a preferred alkaline agent include sodium hydroxide, potassium hydroxide, barium hydroxide, calcium hydroxide, and sodium carbonate.

According to the first embodiment of the manufacturing method, a feed composition is able to be manufactured inexpensively, the feed composition having immunostimulatory action and mold toxin adsorbing action and not decomposing easily. Furthermore, the first embodiment of the manufacturing method facilitates adjustment of the amount of ligno sulfonic acid added. Therefore, the first embodiment is suitably implemented in a case where the amount of ligno sulfonic acid added is desired to be decreased, for example, in a case where the amount is desired to be decreased to about 10 wt%, 8 wt%, or 5 wt% or smaller.

Second Embodiment Related to Manufacturing Method

A second embodiment of the manufacturing method according to the present invention includes:

culturing yeast in a culture medium including ligno sulfonic acid;
subjecting the yeast to alkaline treatment; and
collecting a mixture including the culture medium including ligno sulfonic acid, nucleic acid derived from the yeast, and a yeast cell wall component/yeast cell wall components derived from the yeast.

In this second embodiment of the manufacturing method, ligno sulfonic acid is included in the culture medium where the yeast is cultured. Including ligno sulfonic acid in the culture medium enables minimization of decomposition of yeast during the culturing of the yeast. Furthermore, a feed composition that does not easily decompose is able to be provided because the whole culture medium including ligno sulfonic acid is collected to obtain the feed composition.

The culture medium used in the culturing of the yeast may be obtained by adding ligno sulfonic acid to a culture medium that is generally used for culture of yeast. The lower limit of the content of ligno sulfonic acid to be included in the culture medium may be preferably 1 wt% or higher, more preferably 3 wt% or higher, and even more preferably 5 wt% or higher.

The upper limit of the content of ligno sulfonic acid in the feed composition may be preferably 50 wt% or lower, more preferably 40 wt% or lower, and even more preferably 30 wt% or lower.

When a sufficient amount of yeast has been grown by the culturing, nucleic acid is caused to elute into the culture medium by alkaline treatment of the yeast, similarly to the first embodiment.

After the alkaline treatment, the culture medium including ligno sulfonic acid, the nucleic acid derived from the yeast, and the yeast cell wall components derived from the yeast is collected altogether, and the mixture including the three kinds of components, the nucleic acid, the yeast cell wall components, and ligno sulfonic acid, is thereby able to be obtained. After the alkaline treatment, the pH is preferably adjusted to a pH suitable for feed, before or after the collecting.

The second embodiment enables streamlined manufacture of the feed composition at low cost because the number of processes for obtaining the mixture including the three kinds of components is able to be decreased.

**Examples**

The present invention will hereinafter be described specifically by reference to examples, but the technical scope of the present invention is not limited to the following examples.

The following experiments were conducted to compare and study immunostimulatory effects.

1. Preparation of Feed

The following three types of feed were prepared.

Comparative Example 1 (Feed 1)

An RNA formulation (product name, "RNA-M," manufactured by Nippon Paper Industries Co., Ltd.) was used as Feed 1. "RNA-M" has a molecular weight distribution extending across a range of about 10,000 to 50,000 and is a product including RNAs having a weight-average molecular weight (Mw) of 20,000.

The weight-average molecular weight (Mw) of the nucleic acid may be measured by gel permeation chromatography (GPC). The measurement by GPC may be conducted under the following conditions by a publicly known method using pullulan conversion.

Measurement device: manufactured by Tosoh Corporation
Columns used: Shodex Column OH-pak SB-806HQ, SB-804HQ, and SB-802.5HQ
Eluate: aqueous solution of 1.0% sodium tetraborate and 0.3% isopropyl alcohol
Flow speed of eluate: 1.00 ml/min
Column temperature: 50°C
Concentration of sample measured: 0.2% by mass
Standard material: pullulan (manufactured by Showa Denko K. K.)
Detector: RI detector (manufactured by Tosoh Corporation)
Calibration curve: standardized with pullulan

Comparative Example 2 (Feed 2)

A raw material, yeast (Cyberlindnera jadinii), was cultured using a sulfite pulp waste liquor culture medium having a sugar concentration of 3% and a ligno sulfonic acid concentration of 10% and the yeast was dried with a drum dryer after being collected, thus preparing 100 g of Feed 2.

A literature value, 120,000 (Nippon Kagaku Zasshi, June 1950, Vol. 71, No. 4), was used as the weight-average molecular weight of Feed 2.

Example 1 (Feed 3)

RNA-M and enucleated yeast (product name, "Kabitorula," manufactured by Nippon Paper Industries Co., Ltd.) at a mixture ratio of 1:9 were suspended in water at a concentration of 10 wt% and the water suspension was dried with a drum dryer to prepare 100 g of the obtained mixture, Feed 3. The enucleated yeast of "Kabitorula" includes ligno sulfonic acid.

Nucleic acid in Feed 3 had a weight-average molecular weight (Mw) of 20,000 that was calculated by GPC measurement of a supernatant obtained by centrifugation of the 10% suspension using a high-speed centrifugal machine at 10,000 rpm for ten minutes after stirring of the 10% suspension at 5,000 rpm for 1 hour using a homodisper (homogenizing disperser).

Example 2 (Feed 4)

A raw material, yeast (Cyberlindnera jadinii), was cultured using a sulfite pulp waste liquor culture medium having a sugar concentration of 3% and a ligno sulfonic acid concentration of 10% and the yeast was then collected. Ligno sulfonic acid was contained at 20% by weight. Thereafter, 3000 g of the obtained yeast was stirred in a boiling water bath of 95°C for ten minutes and enzyme in the cells was thereby deactivated. Thereafter, an alkaline extraction reaction was conducted by stirring for two hours after the pH was adjusted to 8.5 with a 48% NaOH aqueous solution in a temperature-adjusted water bath at 55°C. After the reaction, the pH was adjusted to 7.0 with 35% HCl. Thereafter, drying was conducted with a double drum dryer (at a surface temperature of 120°C and 3 rpm), the dried matter that has been formed into a film was ground with a mortar, and a dried product (powder) was obtained. This powder includes nucleic acid derived from the yeast, enucleated yeast cell walls, and ligno sulfonic acid used for the culture. The powder was obtained as Feed 4.

The weight-average molecular weight (Mw) of the nucleic acid in Feed 4 was calculated by a method similar to that for the weight-average molecular weight (Mw) of the nucleic acid in Feed 3 and was found to be 45,000.

Example 3 (Feed 5)

Feed 5 was prepared in the same manner as Example 1 except that an RNA formulation (product name, "RNA-FN," manufactured by Nippon Paper Industries Co., Ltd.) was used for Feed 5. The weight-average molecular weight (Mw) of nucleic acid in Feed 5 was calculated by a method similar to that for the weight-average molecular weight (Mw) of the nucleic acid in Feed 3 and was found to be 11,000.

Contents of soluble nucleic acid, insoluble nucleic acid, magnesium lignosulfonate, and β-glucan in each feed were measured as follows.

Soluble Nucleic Acid and Insoluble Nucleic Acid

Soluble and insoluble nucleic acid obtained by suspending each feed in water were measured according to a quantitative method for polymeric nucleic acid using Schmidt-Thannhauser-Schneider method (see pages 16 to 28 of the 1969 first edition of "Seibutsu-kagaku Jikken-ho by University of Tokyo Press"). "Soluble nucleic acid" is able to be measured by being grouped, by molecular weights, into nucleic acid that are generally relatively high-molecular (for example, having molecular weights ranging from about 10,000 to about 50,000) and nucleic acid that is generally relatively low-molecular (for example, having molecular weights less than about 10,000).

Magnesium Lignosulfonate

A methoxyl group bonded to an aromatic nucleus is generally present in the structure of lignin. The methoxyl group content thus serves as an index of the lignin content. For example, the methoxyl group content was measured by a quantitative method for methoxyl groups by Viebock and Schwappach Method (see pages 336 to 340 of "Methods in Lignin Chemistry" published by Uni Shuppan K. K. in 1994), and the amount of magnesium lignosulfonate was quantitated from the measured methoxyl group content.

β-Glucan

Measurement was conducted using the method described in a β-glucan (β-1,3:-1,6 from yeast) assay kit manufactured by Biocon (Japan) Ltd.

Analytical values for Comparative Examples 1 and 2 and Examples 1 and 2 are listed in Table 1. The unit of the content of each component in Table 1 is wt%.

[Table 1]

TABLE 1: Table of Components

|  | Soluble nucleic acid (%) | Insoluble nucleic acid (%) | Magnesium lignosulfonate (%) | β-glucan (%) |
|---|---|---|---|---|
| Example 1 | 8.0 | 0.4 | 10.0 | 7.0 |
| Example 2 | 8.2 | 0.5 | 20.0 | 8.0 |
| Example 3 | 8.0 | 0.4 | 20.0 | 8.0 |
| Comparative Example 1 | 82.0 | 0.0 | 3.0 | 0.0 |
| Comparative Example 2 | 0.0 | 10.0 | 20.0 | 7.0 |

2. Experimental Groups

The following four experimental groups were set.

Experimental Group 1:

0.0174 mg of Comparative Example 1 (Feed 1)/20 g of body weight (B. W.)

Experimental Group 2:

0.1813 mg of Comparative Example 2 (Feed 2)/20 g of B. W.

Experimental Group 3:

0.1988 mg of Example 1 (Feed 3)/20 g of B. W.

Experimental Group 4:

0.1988 mg of Example 2 (Feed 4)/20 g of B. W.

Control Group:

Physiological saline

Administration of Feed

After introduction of week/weeks-old mice, the mice were randomly divided into groups of five and these groups were each accommodated in a different plastic cage. The mice were then acclimatized to the facility and feed for one week. After the acclimatization, administration of each of the above described types of feed was started. For the experimental groups, the above described types of feed were respectively suspended in physiological saline at the given doses and forcible oral administration was conducted. The same amount of physiological saline was forcibly administered to Control Group for administration of physiological saline. For seven days, the administration was conducted at 4 o'clock in the evening every day.

Collection of Macrophage in Abdominal Cavity

After administering the feed for seven days, 0.4 mL of glycerin solution having a concentration of 1 g/100 mL were injected into the abdominal cavity of each mouse at 5 p.m. and the mouse was kept overnight. The following morning, 5 mL of phosphate buffer saline (PBS) that has been cooled were injected into the abdominal cavity of each mouse, the abdomen was massaged well, and about 4 mL of the intra-abdominal fluid were thereafter taken out with a syringe, placed into a spitz tube, and subjected to centrifugation (at 1200 rpm for five minutes). Washing operation of removing the supernatant and blood cells was conducted twice, the washing operation including: removing the supernatant and red blood cells on the wall surface; thereafter adding cooled PBS and conducting pipetting; and then conducting centrifugation further (at 800 rpm for five minutes). After the washing was completed, suspension was conducted with an RPMI640 culture medium (manufactured by Thermo Fisher Scientific K. K.) added with 10% fetal calf serum (FCS).

Measurement of Phagocytotic Activity of Macrophage for Pathogens

Seeding into a 96-well plate at a macrophage viable cell count of $2 \times 10^5$ cells/mL was conducted and culture was conducted for two hours in a $CO_2$ incubator. After the culture, nonadherent cells were removed, and an antigen (pleuropneumoniae or Streptococcus suis) labeled with fluorescein isothiocyanate (FITC) was added to each of the wells. That is, two types of experimental sections, an experimental section having pleuropneumoniae as the antigen and an experimental section having Streptococcus suis as the antigen, were provided. Furthermore, a blank section not added with the antigen was also provided (not illustrated in FIG. 1). After the culture that was culture implemented with two replicates ended, the number of FITC-positive cells was counted with a flow cytometer. The results are illustrated in FIG. 1.

FIG. 1 illustrates that results related to immune activity are better when nucleic acid is extracted from yeast first and thereafter blended, than when a raw material, yeast, is used as is. That is, the results revealed that a greater immunostimulatory effect is achieved when nucleic acid is extracted from yeast first and thereafter blended back than when a raw material, yeast, is used as is, for a case where both nucleic acid and yeast cell wall components are to be included as active components.

Experiments for Mold Toxin Adsorption Capacity

Mold toxin adsorption capacities of the compositions for feed of Comparative Examples 1 and 2 and Examples 1 and 2 were measured as follows.

Adsorption effects for zearalenone, which is one type of mold toxin, were evaluated as follows. Five milligrams of each specimen were measured into an Eppendorf tube, 0.5 ml of a phosphate buffer for genetic engineering research (1370 mM NaCl, 81 mM $Na_2HPO_4$, 26.8 mM KCl, 14.7 mM $KH_2PO_4$: pH = 7.4) having a zearalenone concentration of 0.0001 mol/L were then placed into the Eppendorf tube and the specimen and the phosphate buffer were mixed well, and the Eppendorf tube was then set in a rotator to be stirred gently for one hour. After each of the compositions was removed by centrifugation, the zearalenone concentration was measured using high-performance liquid chromatography under the following conditions.

Column: Wacosill 5C18RS, 4.6 mm $\times$ 250 mm Column temperature: 40°C
Eluate: methanol/distilled water = 65/35
Injection: 20 $\mu$l
Flow speed: 1 ml/minute
Detection: excitation at 278 nm/measurement at 460nm

The zearalenone adsorption rate (%) was calculated, according to the following equation, from a measured value (A) of the zearalenone concentration before the adsorption treatment and a measured value (B) of the zearalenone concentration after the adsorption treatment.

$$\text{Zearalenone adsorption rate (\%) = \{(A - B)/A\} \times 100}$$

The results are listed in Table 2.

[Table 2]

TABLE 2: Mold Toxin Adsorption Rate

|  | Mold toxin adsorption rate (%) |
|---|---|
| Example 1 | 76.0 |

(continued)

|  | Mold toxin adsorption rate (%) |
| --- | --- |
| Example 2 | 70.0 |
| Example 3 | 75.0 |
| Comparative Example 1 | 2.0 |
| Comparative Example 2 | 63.0 |

Decomposition Resistance Tests

The compositions for feed of Comparative Example 1, Comparative Example 2, Example 1, and Example 2 were respectively placed in laboratory dishes, and left at 35 to 40°C and relative humidity of about 70 to 90% for seven days in a room with a window under a condition where the laboratory dishes were not exposed to direct sunlight.

Foul smells were confirmed for the compositions for feed of Comparative Examples 1 and 2, but foul smells were not generated for Examples 1 and 2.

**Claims**

1.  A feed composition, comprising:

    nucleic acid having molecular weights ranging from 5,000 to 100,000;
    a yeast cell wall component/yeast cell wall components; and
    ligno sulfonic acid.

2.  The feed composition according to claim 1, wherein the content of ligno sulfonic acid ranges from 1 to 30 wt%.

3.  The feed composition according to claim 1 or 2, wherein the nucleic acid is ribonucleic acid derived from yeast.

4.  The feed composition according to any one of claims 1 to 3, further comprising sulfite.

5.  A method of manufacturing a feed composition, the method comprising:
    mixing nucleic acid having molecular weights ranging from 5,000 to 100,000, a yeast cell wall component/yeast cell wall components, and ligno sulfonic acid.

6.  The method of manufacturing a feed composition according to claim 5, comprising:
    collecting nucleic acid and enucleated yeast by separating yeast into the nucleic acid and the enucleated yeast through enucleation of the yeast, thus respectively preparing the nucleic acid having the molecular weights ranging from 5,000 to 100,000 and the yeast cell wall component.

7.  A method of manufacturing a feed composition, the composition comprising nucleic acid having molecular weights ranging from 5,000 to 100,000, a yeast cell wall component/yeast cell wall components, and ligno sulfonic acid, the method comprising:

    culturing yeast in a culture medium including ligno sulfonic acid;
    subjecting the yeast to alkaline treatment; and
    collecting a mixture including: the culture medium including ligno sulfonic acid; nucleic acid derived from the yeast; and a yeast cell wall component/yeast cell wall components derived from the yeast.

[FIG. 1]

## INTERNATIONAL SEARCH REPORT

| International application No. |
|---|
| PCT/JP2021/012817 |

**A. CLASSIFICATION OF SUBJECT MATTER**
A23K 10/16(2016.01)i; A23K 10/30(2016.01)i; A23K 20/153(2016.01)i
FI: A23K20/153; A23K10/16; A23K10/30

According to International Patent Classification (IPC) or to both national classification and IPC

**B. FIELDS SEARCHED**

Minimum documentation searched (classification system followed by classification symbols)
A23K10/00-40/35;50/15, C12N1/00-7/08

| Documentation searched other than minimum documentation to the extent that such documents are included in the fields searched | |
|---|---|
| Published examined utility model applications of Japan | 1922-1996 |
| Published unexamined utility model applications of Japan | 1971-2021 |
| Registered utility model specifications of Japan | 1996-2021 |
| Published registered utility model applications of Japan | 1994-2021 |

Electronic data base consulted during the international search (name of data base and, where practicable, search terms used)
JSTPlus/JMEDPlus/JST7580 (JDreamIII); CAplus (STN)

**C. DOCUMENTS CONSIDERED TO BE RELEVANT**

| Category* | Citation of document, with indication, where appropriate, of the relevant passages | Relevant to claim No. |
|---|---|---|
| X<br>Y | 津田隆治，亜硫酸パルプ排液の利用（発酵生成品およびリグニン），材料, 1967, vol. 16, no. 169, pp. 819-824, page 819, section 2, table II, page 820, section 3.1.2, page 821, left column, lines 5-14, tables IV, VII, page 822, section 3.2, page 823, table XIII, (TSUDA, Ryuzi, "Utilization of Sulfite Waste Liquor (Lignin and Fermented Substance)" Journal of the Society of Materials Science, Japan) | 1, 3-4, 7<br>2, 5-6 |
| X<br>Y | JP 7-184595 A (NIPPON PAPER INDUSTRIES CO., LTD.) 25 July 1995 (1995-07-25) paragraphs [0005]-[0009] | 1, 3-4<br>2, 5-6 |

☒ Further documents are listed in the continuation of Box C.    ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 28 May 2021 (28.05.2021) | 15 June 2021 (15.06.2021) |

| Name and mailing address of the ISA/<br>Japan Patent Office<br>3-4-3, Kasumigaseki, Chiyoda-ku,<br>Tokyo 100-8915, Japan | Authorized officer<br><br>Telephone No. |
|---|---|

Form PCT/ISA/210 (second sheet) (January 2015)

**INTERNATIONAL SEARCH REPORT**

International application No.

PCT/JP2021/012817

C (Continuation).   DOCUMENTS CONSIDERED TO BE RELEVANT

| Category* | Citation of document, with indication, where appropriate, of the relevant passages | Relevant to claim No. |
|---|---|---|
| Y | 賦形物質リグノスルホン酸カルシウム及びリグニンスルホン酸ナトリウム，食品安全委員会, 2012, page 4, abstract, non-official translation ("Calcium lignosulfonate and sodium lignosulfonate as excipients", Food Safety Commission of Japan) | 2, 5-6 |
| Y | 日本製紙（株）江津工場，紙パ技協誌, 1998, vol. 52, no. 6, pp. 838-847, page 842, left column, line 6 to page 843, right column, line 2, non-official translation (NIPPON PAPER INDUSTRIES CO., LTD., "Gotsu factory", JAPAN TAPPI JOURNAL) | 6 |
| A | JP 9-327268 A (KIKKOMAN CORPORATION) 22 December 1997 (1997-12-22) entire text, all drawings | 1-7 |
| A | CN 101480220 A (JIANGSU INSTITUTE OF SUWEI MICROBIOLOGY RESEARCH) 15 July 2009 (2009-07-15) entire text, all drawings | 1-7 |

Form PCT/ISA/210 (continuation of second sheet) (January 2015)

**INTERNATIONAL SEARCH REPORT**

Information on patent family members

| International application No. |
|---|
| PCT/JP2021/012817 |

| Patent Documents referred in the Report | Publication Date | Patent Family | Publication Date |
|---|---|---|---|
| JP 7-184595 A | 25 Jul. 1995 | (Family: none) | |
| JP 9-327268 A | 22 Dec. 1997 | (Family: none) | |
| CN 101480220 A | 15 Jul. 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H07184595 A
- JP 6530846 B
- JP 2002512011 A

**Non-patent literature cited in the description**

- *Nippon Kagaku Zasshi,* June 1950, vol. 71 (4